# EUROPEAN PATENT SPECIFICATION

(11) **EP 1 099 432 B1**
(45) Date of publication and mention of the grant of the patent: **13.04.2005**
(21) Application number: 00118816.8
(22) Date of filing: 31.08.2000
(51) Int. Cl.: A61F 9/007

(54) **A surgical swivel fixation ring device for use in eye surgery**
Chirurgischer Schwenkhaltering zur Verwendung in der Augenchirurgie
Anneau chirurgical de fixation pivotant pour l'utilisation en chirurgie de l'oeil

(30) Priority: 10.11.1999 US 437777
(43) Date of publication of application: 16.05.2001
(73) Proprietor: Becton Dickinson and Company, Franklin Lakes, New Jersey 07417 (US)
(72) Inventor: Cote, Dana, Saugus, Massachusetts 01906 (US)
(74) Representative: Ruffles, Graham Keith

(56) References cited:
- DE-A- 19 500 708
- US-A- 5 108 412
- US-A- 5 951 579

## Description

### 1. FIELD OF THE INVENTION

The field of invention relates to a medical surgical device for stabilizing the eye during surgery. More particularly, the invention relates to a swivel fixation ring for use in corneal surgery.

### 2. BRIEF DESCRIPTION OF THE PRIOR ART

In the medical profession, cataract extraction and lens implantation surgery has evolved significantly resulting in safer and more predictable outcomes. A surgical technique called the clear corneal approach was developed that offered significant advantages over the previous surgical technique for cataract surgery. In this technique, the primary incision is made only through the corneal tissue.

In cataract surgery, a high degree of precision is required to insure the surgical outcome will be a success. A major concern is location of the incision. The incision geometry must be properly sized and located to diminish the chances of complication. Complications include, for example, leakage of the ocular fluid, corneal bum or increasing a pre-operative stigmatism. A properly designed scalpel and experienced surgeon are both important in achieving a successful incision. During surgery, the patient is fully alert and can inadvertently move his or her eyes during advancement of the scalpel into the eye. This can cause the sharp edges of the scalpel to uncontrollably widen the incision greater than the width of the scalpel. This widening of incision can lead to unanticipated complications. Another potential risk during eye movement is the possibility of incising other intraocular tissues such as the iris or the lens.

Surgeons are well aware of the problems and employ a number of tools to stabilize the eye during surgery. These can be as simple as the surgeon's fingers or forceps pressing on the opposite side of the eye from the incision. The purpose is to cancel out any motion that is created by the cutting forces of the scalpel. Typically, these methods have had little effect on inhibiting patient's eye movement during surgery. A number of devices have been manufactured to solve this problem. These devices generally have a semi-circular ring with or without a series of teeth to embed in the eye where the ring is attached to the handle. The surgeon holds the handle and depresses the ring against the sclera of the eye. This ring device generally wraps around the sclera leaving an opening adequate to manipulate instruments which would include the scalpel.

Current commercial surgical fixation rings are usually reusable and made of stainless steel or titanium. These devices can be very expensive due to the exotic material that they are made out of. A common design for such a prior art device contains a ring attached to the distal end of the handle fixed at a pre-determined angle. A second common type of design for such a surgical fixation ring device has the distal end of the handle with a hinge mechanism that allows the ring to swivel at a variety of angles. This swivel design uses multiple components usually a handle welded to an integral fork, a set of two pins and a separate ring. The swivel design is usually preferred over the stationary design in that it can adapt to a variety of positions that provides stabilization of the eye and the ability to clear facial features such as the nose or brow.

There still remains several problems when utilizing these prior art devices. For example, the swivel fixation ring of the prior art design still has a high probability of slipping off the sclera causing the eye to move and potential trauma due to movement of the eye during the initial incision. In addition, when teeth are employed in the prior art design the teeth tend to cause damage to the sclera and corneal tissue as they cut into the tissue in trying to hold firmly on the eye to reduce slipping. Prior art designs that incorporate multiple ribs or grooves cause irritation to the eye due to pinpoint contact on the sclera or corneal tissue.

Thus, there remains a need for a swivel fixation ring that will allow the surgeon to have more surface contact with the eye to assist and secure fixation of the ring during the primary incision. In addition, there is a need for a fixation device to have a smaller overall diameter that will allow more clearance between the ring and other facial obstructions so that the surgeon can use other surgical instruments on the eye without interference due to the fixation ring. Another improvement needed for the fixation ring device is that the teeth must always be in the correct orientation pointing down. Some prior art swivel-type fixation rings that contain teeth on the bottom tend to point up and away from the scleral surface during use. There remains a need for a device that can correct this deficiency and have teeth that are in the correct orientation pointing toward the sclera. There is also a need for teeth design that cause less of a trauma to the sclera and corneal tissue. Finally, there is a long felt need for a fixation ring device that not only reduces costs but also has the ability to be readily cleaned. Some prior art devices of the swivel-type design contain pins which create small crevices between parts that are very difficult to clean. There is a need to have a device to make it easier to clean and reuse.

A device having the features of claim 1, except for an arcuate bottom and a living hinge, is taugth by US 5,951,759.

### 3. SUMMARY OF THE INVENTION

The present invention avoids a disadvantage to the prior art by allowing the interior ring surface to match with the curvature of the sclera. This matching of surfaces gives the assistance to secure more tightly to the surface of the sclera without trauma as in the other prior art devices. Another objective that is met in the present invention is that due to the nature of the inherent living hinge design, the protrusions or teeth attach to the bottom surface of the ring can grasp the sclera thereby decreasing slippage without producing additional trauma to the eye. The living hinge also assists in eliminating the problem of the ring swiveling away from the scleral surface during use as can happen with the prior art device. The third object of the present invention is that the overall diameter is smaller by incorporating the living hinges mounted on top of the ring. This smaller diameter assists the surgeon by giving more clearance between the ring and other facial obstructions. Additionally, the device of the present invention eliminates small crevices which were present in the pin section that are hard to clean during re-use.

Accordingly, there is provided in the present invention, according to independent claim 1, a surgical fixation ring device for placement on the sclera during eye surgeries including a handle having a handle base, a shaft and a u-shaped fork. The handle is attached to a segmented ring base that has an arcuate bottom. The arcuate bottom is designed to mate with the curvature of the sclera of the eye. Further included is a living hinge attached between the handle and the segmented ring base for allowing the ring base to swivel relative to the handle about 180°. The ring base further includes protrusions or teeth that are used to anchor or eliminate slippage of the ring base from the sclera. The ring base is segmented. In a preferred embodiment, it wraps around the cornea from about 180° to about 270°. This design allows maximum surface contact of the arcuate bottom to the sclera to decrease slippage on the sclera and allows an opening adequate to manipulate surgical instruments such as the scalpel.

### 4. BRIEF DESCRIPTION OF THE DRAWINGS

Fig. 1 is a perspective view of a surgical fixation ring device of the prior art.
Fig. 2 is an enlarged side view of the prior art device in Fig. 1 illustrating relative motion of the fixation ring.
Fig. 3 is a perspective view of the surgical fixation ring device in accordance with the subject invention.
Fig. 4 is an enlarged view of Fig. 3 illustrating the fixation ring in accordance with the subject invention.
Fig. 5 is an enlarged side view of the device in Fig. 3 demonstrating relative motion of the fixation ring in accordance with the subject invention.
Fig. 6 is an enlarged perspective view of the device in Fig. 3 in relation to a human eye.
Fig. 7 is the device illustrated in Fig. 6 placed on the sclera.
Fig. 8 is a perspective view of a prior art device placed on the sclera.
Fig. 9 is a cross-sectional view of the present invention taken along lines 9-9 in Fig. 7.
Fig. 10 is an enlarged cross-sectional view of Fig. 9.
Fig. 11 is a cross-sectional view of a prior art device taken along lines 11-11 in Fig. 8.
Fig. 12 is an enlarged cross-sectional view of Fig. 11.
Fig. 13 is a front view of the present invention relative to the sclera.
Fig. 14 is a front view of a prior art device relative to the sclera.

### 5. DETAILED DESCRIPTION

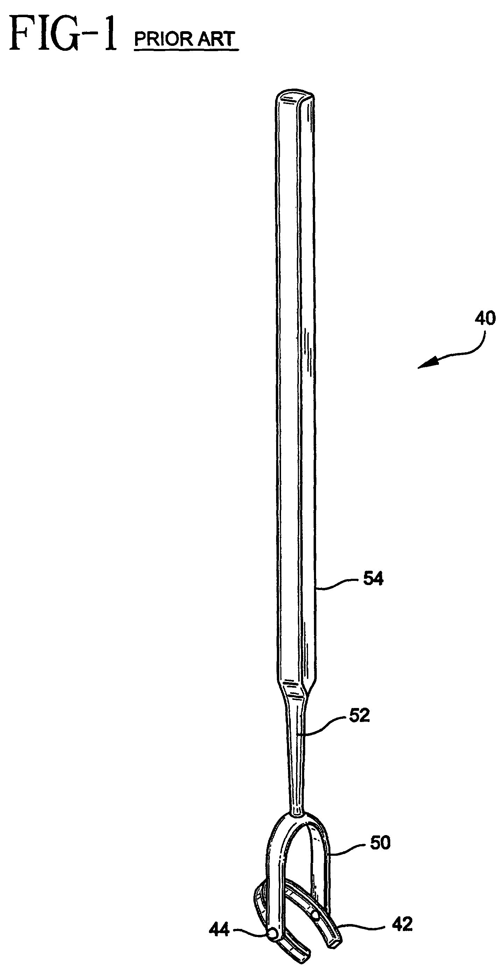

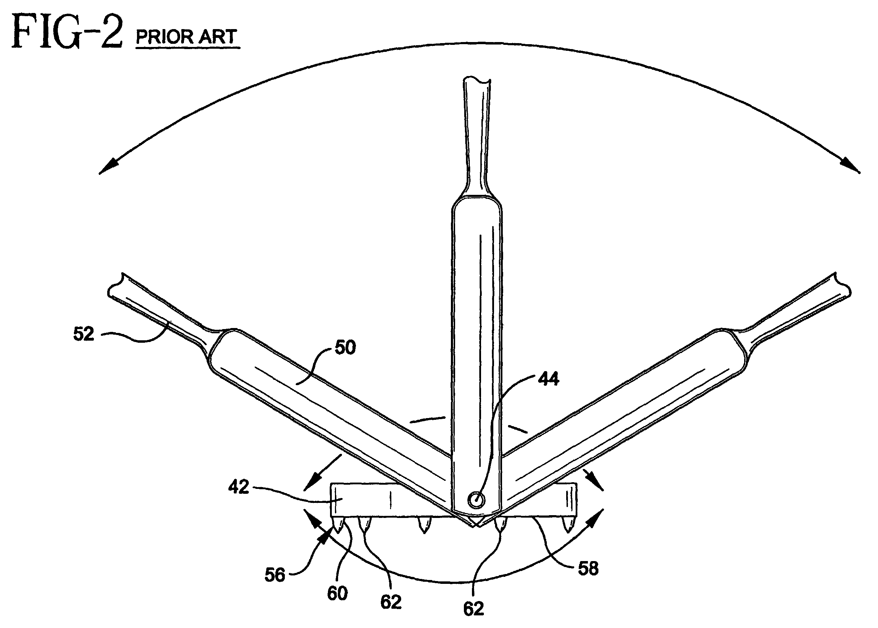

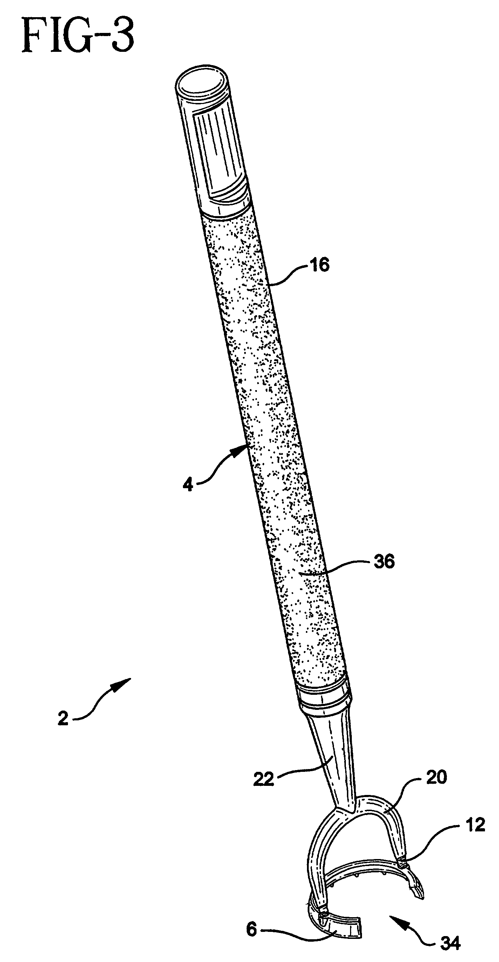

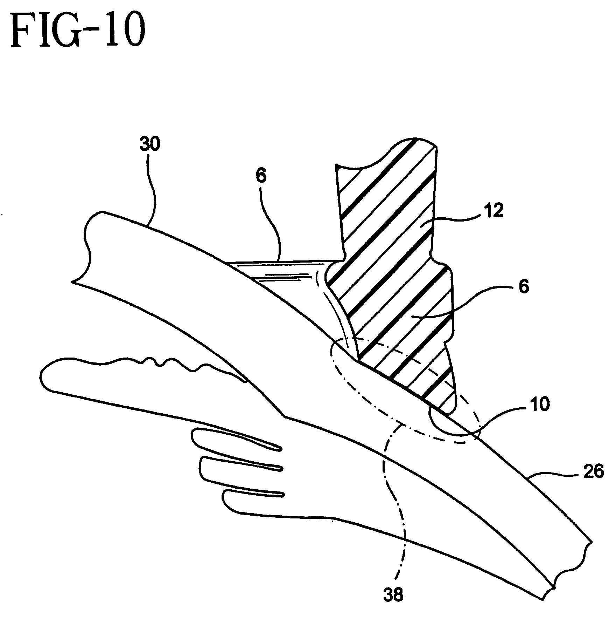

A surgical fixation ring device for placement on the sclera of an eye during surgery in accordance with the subject invention is identified generally by the numeral **2** in Figs. 3-14. Figs. 1-2 illustrate a prior art fixation ring device generally identified by the numeral **40**. Device **40** includes a handle **54**, a shaft **52**, a u-shaped fork **50**, a ring **42**, and a plurality of pins **44**. Handle **54** is connected to shaft **52** which is connected to u-shaped fork **50**. U-shaped fork **50** is disposed over ring **42** and attached to ring **42** by pins **44**. This configuration allows ring **42** to rotate 360° relative to fork **50** as illustrated in Fig. 2. Ring **42** further includes a flat bottom **58** which includes teeth **56**. Teeth **56** further includes a plurality of spikes **60** that are disposed circumferentially around ring **42**. Spike **60** form a sharp point **62**.

Adverting to the drawings, Figs. 1 and 2 illustrate the prior art fixation ring device. Ring **42** is able to rotate relative to pin **44** 360°. Thus, the ring can swivel during handling and surgery which can make the ring point up and away from the scleral surface. This movement can force the doctor to stop surgery and possibly drop another instrument to manually rotate the ring back into the position to hold onto the scleral surface. Spike **60** contains sharp point **62** which can cause traumatic tissue damage to the sclera when embedded in the scleral surface. The handle is allowed to rotate 360° relative to the pin around the ring. Again this feature has a disadvantage of the handle and the ring swiveling out of place during surgery which would require the surgeon to reposition the device on the scleral surface.

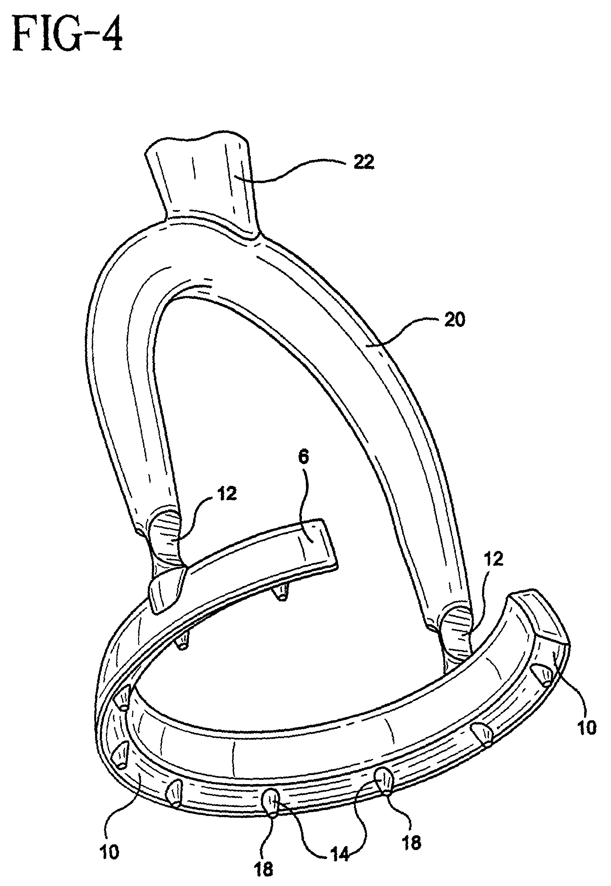

Figs. 3-5 illustrate the present invention of the surgical fixation ring. Shown in Figs. 3 and 4 is surgical fixation ring **2**. Surgical fixation ring **2** includes a handle assembly **4** attached to a segmented ring base **6**. Handle assembly **4** preferably includes a handle base **16**, a shaft **22**, and a fork **20**. Handle base **16** further includes a gripping surface **36**. Gripping surface **36** is preferably textured or embossed such that the surgeon is easily able to grasp the device without any slippage of the device from the surgeon's hand. The shaft and fork are preferably a unitary structure, however it does not have to be so designed. The handle shaft and fork are also preferably a unitary structure. However, it is within the scope of the invention to have a handle that allows the shaft and fork to be replaceable. In such an embodiment, the handle would include mating structures to connect to the shaft. Such structures would be known to those skilled in the art and include snap-fits, threads, and other types of fasteners. The fork is preferably U-shaped however it does not have to be so shaped. Other typical shapes could be used for example a V-shaped or even a single unitary structure such as an arm to the shaft connecting directly to the ring. Additionally, the fork can be eliminated from the present invention and the shaft can be directly connected to the segmented ring.

Preferably, the surgical fixation ring device is a unitary structure. This would include that the handle assembly and the segmented ring base are a unitary structure. However, it is within the scope of this invention, that each individual component can be removably attached from each other. For example, the handle can be removably attached from the shaft, the shaft can be removably attached from the fork, and the fork can be removably attached from the segmented ring. This attachment replaceability would enable the surgeon to attach various different fixation rings on the device which would give the surgeon more flexibility during the eye surgery. Additionally, it is within the scope of the invention to have the handle assembly only comprise a handle and a shaft which would be attached to the segmented ring. It is also within the scope of the invention for the handle to be attached to the segmented ring.

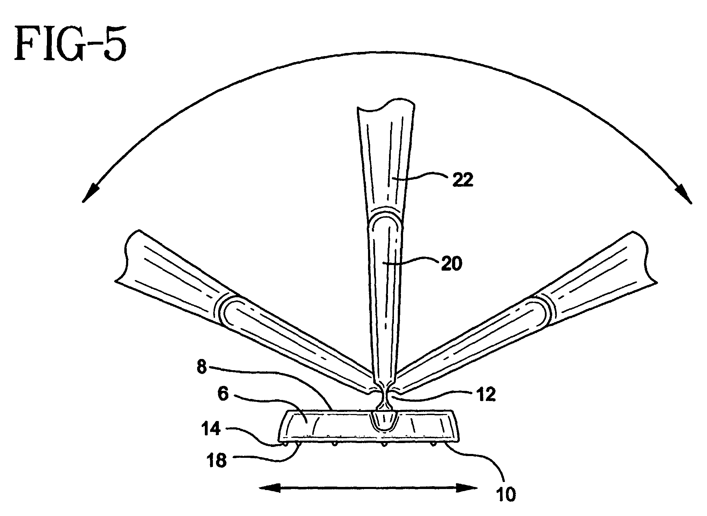

Adverting to Fig. 4, segmented ring base **6** is attached to the handle assembly by at least one living hinge **12**. Living hinge **12** is preferably attached on the segmented ring such that the distal end of the living hinge is attached to the top of the segmented ring as shown in Fig. 4. This position provides the many advantages to the present invention. For example, the segmented ring is unable to rotate 360° as shown in Fig. 5 due to the position of the living hinge. Unlike the prior art shown in Fig. 2, the present invention provides the surgeon with a fixation ring that will not flip away from the scleral surface such as the prior art device would be prone to do.

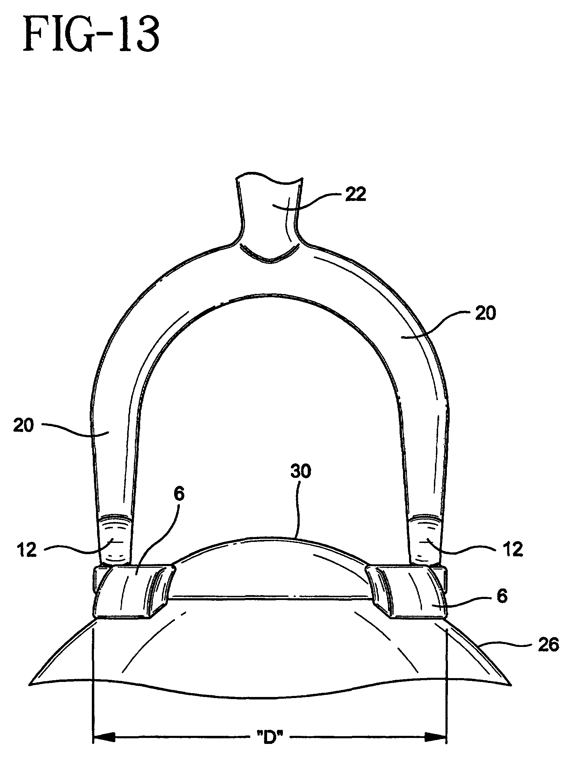

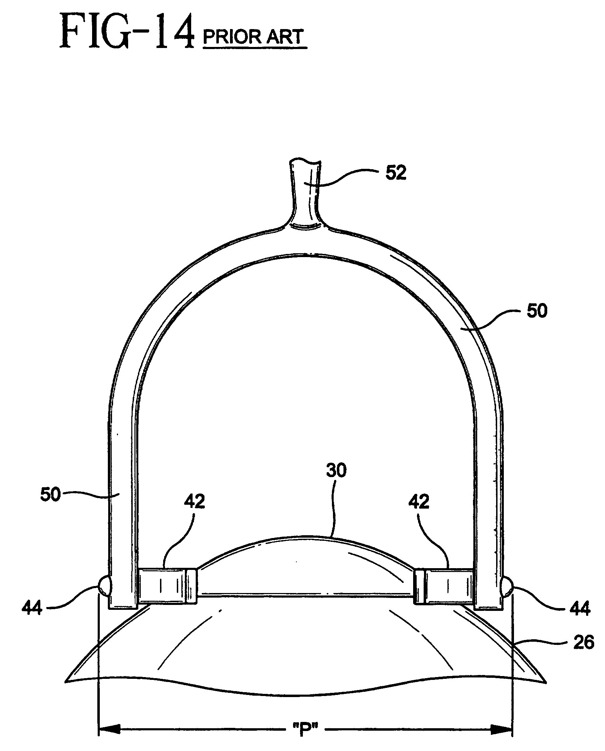

Additionally, the position of the living hinge provides a working diameter **"D"** as shown in Figs. 13 and 14 that is smaller than the working diameter of the prior art device **"P"**. Working diameter **"D"** is defined as the width of the segmented ring which would include any fasteners or attachments to connect this ring to the handle assembly. As shown in Fig. 13, working diameter **"D"** is smaller than working diameter **"P"**. This smaller width assists in giving the surgeon more clearance between the ring and other facial obstructions. Thus, the surgeon has more area to work during the eye surgery. By the prior art device placing the attachment to the segmented ring on the side of the segmented ring, the width working diameter **"P"** is increased. This structural feature would be a disadvantage to the surgeon because the surgeon would have less of a working area during the surgery of the eye. Preferably, living hinge **12** is made of polypropylene. Covered within the scope of this invention that the living hinge and surgical fixation device could be made of a thermoplastic material selected from the group consisting of polyethylene, polypropylene, nylon, polyvinyl chloride, polystyrene, thermoplastic elastomers, natural rubber and combinations thereof. However, preferably, the device is made of a thermoplastic material such as polypropylene. Thus, the living hinge attached to the handle assembly provides for rotation of the handle base relative to the ring base of about 180° of rotation.

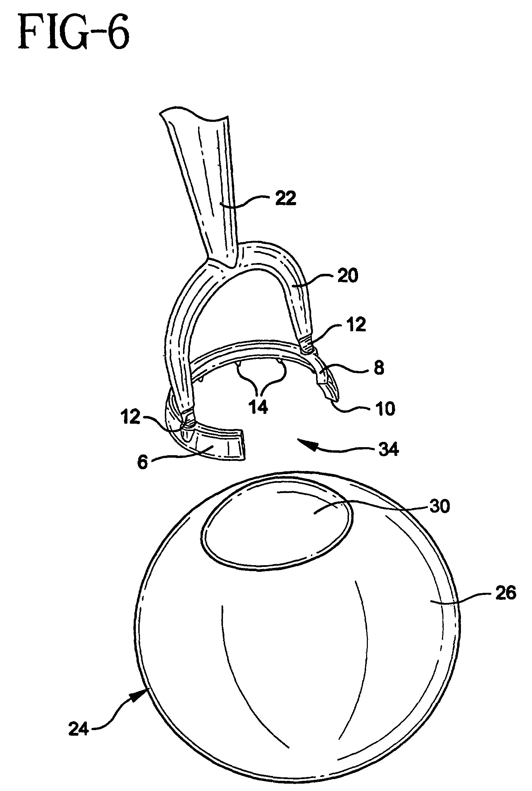

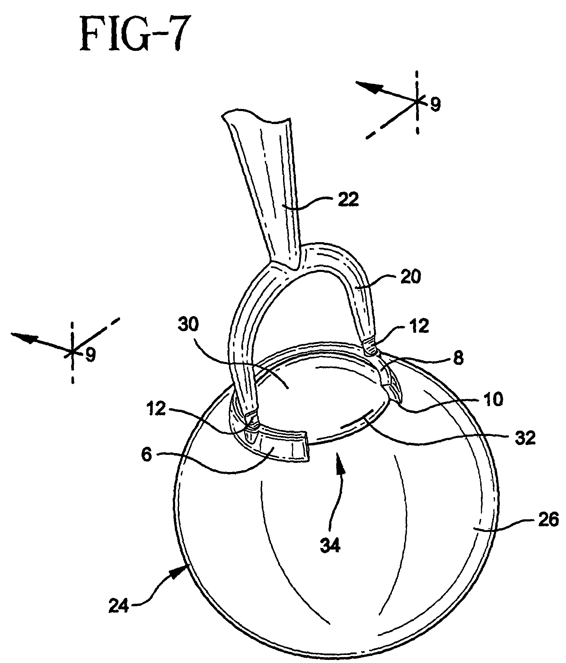

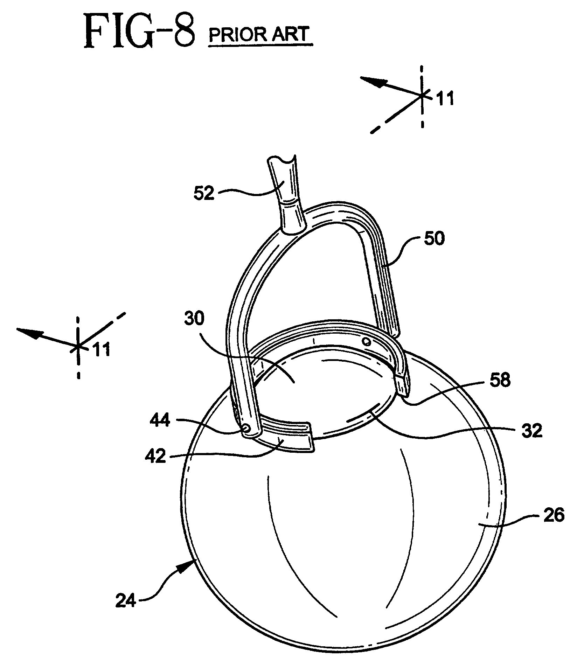

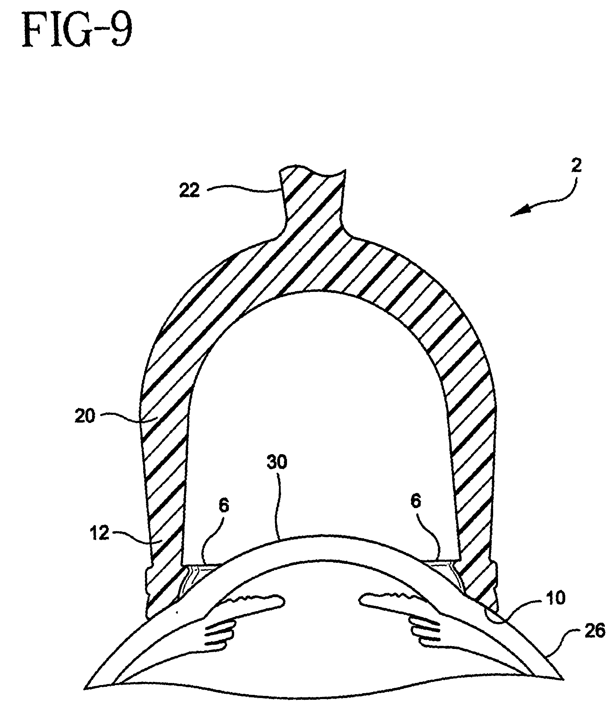

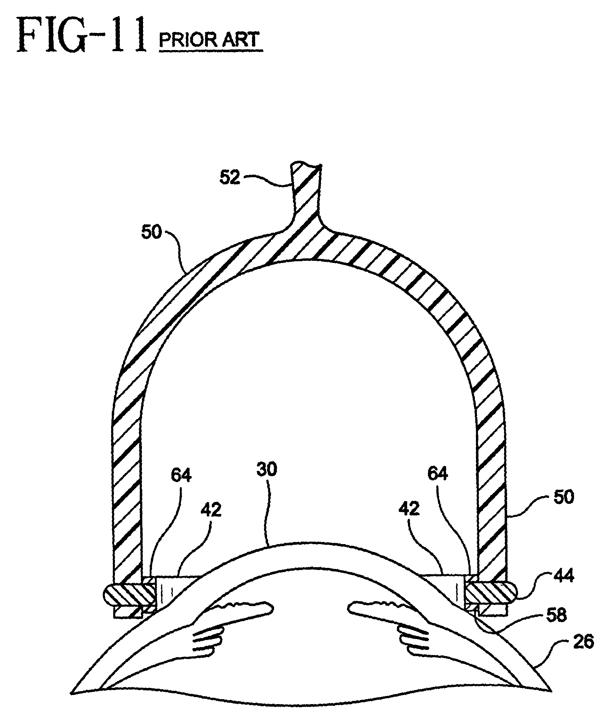

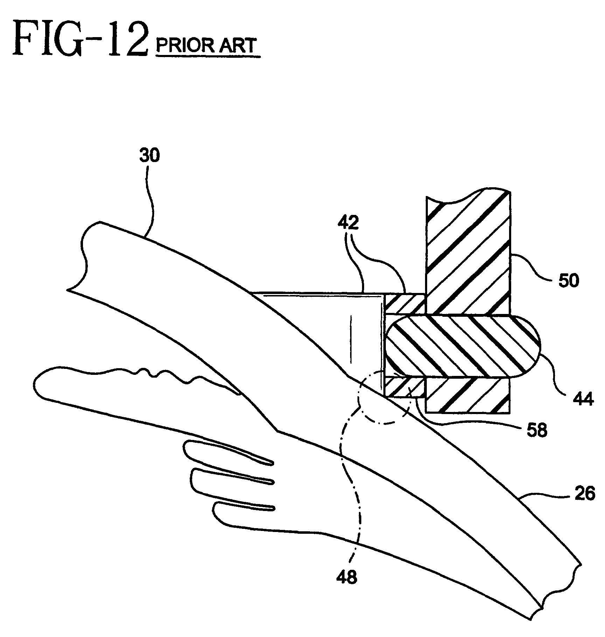

Segmented ring base **6** further includes an arcuate bottom **10** that is designed to mate with the curvature of eye **24** and in particular sclera **26**. As shown in Figs. 6 and 7, eye **24** composes a sclera **26** and a cornea **30**. The fixation ring device is seated over the cornea onto the sclera. Thus, arcuate bottom **10** has a curvature that mates with the curvature of the sclera to provide more contact surface with the fixation ring to decrease the potential of slippage of the device. Preferably, the segmented ring device has a circumference of between about 180° to 270° relative to the sclera. This allows the ring base to wrap around the sclera and provide an open end **34** that allows the surgeon to place an incision **32** in the cornea. Unlike the prior art device that is shown in Fig. 8 arcuate bottom **6** allows a less traumatic exposure to the scleral tissue because of the mating surface of bottom **10** to the sclera as shown in Fig. 8 and more particularly, in Figs. 9-12. The present invention allows a contact surface **38** that mates with sclera **26** to provide less trauma to the eye and more contact surface to the device to prevent slipping. As shown in Figs. 11 and 12, the prior art device has a contact surface **48** which is cornered or pinpointed into sclera **26**. This corner can cause trauma to the sclera as well as increasing the potential of slippage of the fixation ring by having a small contact surface of the prior art fixation ring to the sclera.

In addition, segmented ring base **6** preferably includes a plurality of protrusions **14** attached to arcuate bottom **10**. The purpose of the protrusions is to atraumatically grip onto the sclera thereby further decreasing the potential of the fixation ring to slip off of the eye during surgery. Preferably, the protrusions are made of a thermoplastic material selected from the group consisting of polyethylene, polypropylene, nylon, polyvinyl chloride, polystyrene, thermoplastic elastomers, natural rubber, and combinations thereof. Preferably, the ring base includes the working diameter of between about 12mm to about 14mm and in particular has a working diameter about 14.75mm. Additionally, the protrusions are conically shaped and have a radius bottom point **18**. Radius point **18** allows the fixation ring to atraumatically attach to the sclera unlike the prior art device which has a spike **60** and sharp point **62** as shown in Fig. 2 that can cause trauma to the sclera when the prior art device is attached. The open end is dimensioned wide enough to insert a scalpel to incise the eye. However, the ring base is preferably designed so that the circumference of the ring base is between about 180° to 270° relative to the sclera so that the ring base maximizes surface contact to the sclera thereby reducing the potential for the fixation ring to slip off of the eye.

Living hinge **12** also give another advantage to the fixation ring device such that if the device is to be reused, cleaning the device is made much easier by eliminating the need to clean small crevices. As shown in Figs. 9 and 11, a cross-sectional view of the device relative to the eye shows that pins **44** of the prior art device forms a small crevice **64** in between the pin and the prior art fixation ring. The fixation ring of the present invention eliminates the crevice by having the living hinge connected to the top of the ring base.

Additionally, surgical fixation ring device **2** has the advantage of providing an adjustable swivel fixation ring **6** with the above features that can be manufactured with efficiency and thus lower cost than the other prior art fixation ring devices. The surgical fixation ring **2** of the present invention can be used in any eye surgery requiring holding of the eye.

The surgical fixation ring **2** can also have handle assembly **4** with various components. Handle assembly **4** could just include handle **16** attached to segmented ring **6**. To provide for the swivel feature, living hinge **12** would be attached to handle **16** and fixation ring **6**. Additionally, handle assembly **4** could include handle **16** and shaft **22** attached to segmented ring **6**. Again, for the swivel feature, living hinge **12** would be attached to shaft **22** and fixation segmented ring base **6**. Lastly, handle assembly **4** could include handle **16** attached to shaft **22** which would be attached to a fork **20**. Fork **20** would be attached to segmented ring **6** and for providing the swivel feature a living hinge would connect fork **20** to segmented ring **6**. This last embodiment would be the preferred embodiment which would allow the segmented ring **6** to swivel to about 180° relative to the handle base relative to the ring base. Again, prior art devices have the disadvantage of having the prior art ring base rotate 360° relative to the handle base which gives the potential of the ring base rotating away from the scleral tissue. Portions of the handle assembly can be removably attached to allow the surgeon the flexibility of using various geometries of the ring base thereby giving the surgeon flexibility during the eye surgery. The operation and function of the surgical fixation ring device **2** would include the following. The fixation ring is designed to stabilize the eye when the corneal marker or knives are being used on the eye. The fixation ring has the advantage of stabilizing the eye without incurring any potential trauma to the eye and maximizing surface contact between the fixation ring and the eye. The first step in the procedure is to place the fixation ring using a non-dominant hand with the open end of the ring base overlying the site of the intended incision. The eye is pressed down by applying force on the handle assembly **4** by gently downward pressure applied to the sclera. Repositioning of the handle assembly is allowed by swiveling the handle assembly relative to the fixation ring by the flexibility of the living hinge. Thus, the handle assembly can be moved to various positions relative to the fixation ring while the fixation ring continually holds the eye in place. This feature allows the surgeon to maximize his working area while maintaining fixation of the eye. Surface contact between the fixation ring and the sclera is maximized by the arcuate bottom mating with the curvature of the sclera. Potential slippage of the fixation ring is further reduced by having a plurality of protrusions attached to the arcuate bottom. Further trauma to the eye is decreased by having the protrusions conically designed with a bottom radius point **18**. The fixation ring may be clamped by the handle assembly to allow the surgeon to have use of both hands during the eye surgery.

The following steps outline how the device is typically used during eye surgery. The surgeon determines the appropriate location to create the primary incision. The segmented ring base is oriented concentric to the cornea with the open end of the ring base located adjacent to the primary incision location. The surgical fixation ring is then lowered until the arcuate bottom is depressed against the scleral surface thereby embedding the protrusions located on the arcuate bottom. The surgeon can pivot the handle at any time to any comfortable position while maintaining eye fixation. The fixation ring stabilizes the eye so the surgeon can make primary incisions or any other type of incisions as well as insertion or removal of lens before suturing or any type of surgical procedure required. After the surgical procedure, the device is lifted off so that the eye is able to move.

The embodiments depicted in the figures are intended to be merely exemplary and not intended to depict all possible surgical fixation ring devices. Rather, surgical fixation ring **2** can have any structure that allows the handle assembly to pivot relative to the segmented ring base to about 180°. The ability to control the swiveling or pivoting of the segmented ring base relative to the handle and by providing a contoured or arcuate bottom to the segmented ring base as well as the addition of an atraumatic protrusion design on the arcuate bottom to decrease slippage of the ring base greatly facilitates the use of the surgical fixation ring to position the eye during surgery without causing major trauma to the eye and reducing slippage of the fixation ring device.

## Claims

1. A surgical fixation ring device for placement on the sclera during eye surgery, comprising:
a handle assembly having a handle base, a shaft and a U-shaped fork;
a segmented ring base having a top, a working diameter, an open end and an arcuate bottom designed to mate with the curvature of the sclera for reducing slippage of said ring base, said open end dimensioned wide enough for insertion of a scalpel therethrough to incise the eye;
a living hinge attached to said top and said fork for rotation of said ring base relative to said fork; and
a plurality of protrusions circumferentially disposed around said arcuate bottom to atraumically grip the sclera.

2. The device in Claim 1 wherein said segmented ring base has a circumference of between about 180° to 270° relative to the sclera.

3. The device in Claim 1 wherein said ring base further includes a working diameter of between about 12mm to about 16mm.

4. The device in Claim 3, wherein said working diameter is about 14.75mm.

5. The device in Claim 1, wherein said shaft, said U-shaped fork, said ring base, said living hinge, and said protrusions are a unitary structure of thermoplastic material.

6. The device in Claim 5, wherein said shaft is detachably connected to said handle base.

7. The device in Claim 6 wherein said handle assembly, said living hinge and said ring base are made of a thermoplastic material.

8. The device in Claim 1, wherein said protrusions are conical-shaped and have a bottom radius.

9. The device in Claim 1, wherein said protrusions are made of a thermoplastic material selected from the group consisting of polyethylene, polypropylene, nylon, polyvinyl chloride, polystyrene, thermoplastic elastomers, natural rubber, and combinations thereof.

10. The device in Claim 9, wherein said handle base further comprises a gripping surface.

## Patentansprüche

1. Chirurgische Halteringvorrichtung für eine Anordnung auf der Sklera während der Augeuchirurgie, die aufweist:
eine Griffbaugruppe mit einer Griffbasis, einem Schaft und einer U-förmigen Gabel;
eine segmentierte Ringbasis mit einer Oberseite, einem Arbeitsdurchmesser, einem offenen Ende und einem bogenförmigen Boden, so konstruiert, daß eine Anpassung an die Krümmung der Sklera für das Verringern des Rutschens der Ringbasis zu verzeichnen ist, wobei das offene Ende breit genug füt das Einsetzen eines Skalpells dort hindurch dimensioniert ist, um das Auge aufzuschneiden;
ein bewegliches Gelenk, das an der Oberseite und der Gabel für eine Drehung der Ringbasis relativ zur Gabel befestigt ist; und
eine Vielzahl von Vorsprüngen, die peripher um den bogenförmigen Boden angeordnet sind, um die Sklera atraumatisch zu erfassen.

2. Vorrichtung nach Anspruch 1, bei der die segmentierte Ringbasis einen Umfang von zwischen etwa 180° bis 270° relativ zur Sklera aufweist.

3. Vorrichtung nach Anspruch 1, bei der die Ringbasis außerdem einen Arbeitsdurchmesser von zwischen etwa 12 mm und etwa 16 mm umfaßt.

4. Vorrichtung nach Anspruch 3, bei der der Arbeitsdurchmesser etwa 14,75 mm beträgt.

5. Vorrichtung nach Anspruch 1, bei der der Schaft, die U-förmige Gabel, die Ringbasis, das bewegliche Gelenk und die Vorsprünge eine unitäre Konstruktion aus thermoplastischem Material sind.

6. Vorrichtung nach Anspruch 5, bei der der Schaft lösbar mit der Griffbasis verbunden ist.

7. Vorrichtung nach Anspruch 6, bei der die Griffbaugruppe, das bewegliche Gelenk und die Ringbasis aus einem thermoplastischen Material hergestellt werden.

8. Vorrichtung nach Anspruch 1, bei der die Vorsprünge kegelförmig sind und einen Basisradius aufweisen.

9. Vorrichtung nach Anspruch 1, bei der die Vorsprünge aus einem thermoplastischen Material hergestellt werden, das aus der Gruppe ausgewählt wird, die besteht aus: Polyethylen; Polypropylen; Nylon; Polyvinylchlorid; Polystyrol; thermoplastischen Elastomeren; Naturkautschuk; und deren Kombinationen.

10. Vorrichtung nach Anspruch 9, bei der die Gxiffbasis außerdem eine Grifffläche aufweist.

## Revendications

1. Anneau chirurgical de fixation destiné à être placé sur la sclérotique au cours de la chirurgie de l'oeil, comprenant:
un assemblage de manche comportant une base de manche, une tige et une fourche en U;
une base segmentée de l'anneau comportant une partie supérieure, un diamètre de travail, une extrémité ouverte et une partie inférieure arquée destinée à s'adapter à la courbure de la sclérotique pour réduire le glissement de ladite base de l'anneau, ladite extrémité ouverte étant suffisamment grande pour permettre l'insertion d'un scalpel en vue d'une incision de l'oeil;
une charnière active fixée sur ladite partie supérieure et ladite fourche pour permettre une rotation de ladite base de l'anneau par rapport à ladite fourche; et
plusieurs saillies agencées circonférentiellement autour de ladite partie inférieure arquée pour permettre une saisie non traumatique de la sclérotique.

2. Dispositif selon la revendication 1, dans lequel ladite base segmentée de l'anneau a une circonférence comprise entre environ 180° et 270° par rapport à la sclérotique.

3. Dispositif selon la revendication 1, dans lequel ladite base de l'anneau englobe en outre un diamètre de travail compris entre environ 12 mm et environ 16 mm.

4. Dispositif selon la revendication 3, dans lequel ledit diamètre de travail est de l'ordre de 14,75 mm.

5. Dispositif selon la revendication 1, dans lequel ladite tige, ladite fourche en U, ladite base de l'anneau, ladite charnière active et lesdites saillies constituent une structure d'une seule pièce composée d'un matériau thermoplastique.

6. Dispositif selon la revendication 5, dans lequel ladite tige est connectée de manière amovible à ladite base du manche.

7. Dispositif selon la revendication 6, dans lequel ledit assemblage de manche, ladite charnière active et ladite base de l'anneau sont composés d'un matériau thermoplastique.

8. Dispositif selon la revendication 1, dans lequel lesdites saillies ont une forme conique et un rayon de base.

9. Dispositif selon la revendication 1, dans lequel lesdites saillies sont composées d'un matériau thermoplastique sélectionné dans le groupe constitué de polyéthylène, de polypropylène, de nylon, de chlorure de polyvinyle, de polystyrène, d'élastomères thermoplastiques, de caoutchouc naturel et de combinaisons correspondantes.

10. Dispositif selon la revendication 9, dans lequel ladite base du manche comprend en outre une surface de préhension.
